# EUROPÄISCHE PATENTSCHRIFT

(11) **EP 1 425 296 B1**
(45) Veröffentlichungstag und Bekanntmachung des Hinweises auf die Patenterteilung: **13.04.2011**
(21) Anmeldenummer: 02797977.2
(22) Anmeldetag: 10.09.2002
(51) Int. Cl.: C07K 7/06, A61K 38/04

(54) **ORGANISCHE VERBINDUNGEN MIT BIOLOGISCHER WIRKUNG ALS THROMBINHEMMER UND IHRE VERWENDUNG**
ORGANIC COMPOUNDS WITH BIOLOGICAL ACTIVITY AS THROMBIN INHIBITORS AND USE THEREOF
COMPOSES ORGANIQUES A ACTIVITE BIOLOGIQUE UTILISES COMME INHIBITEURS DE THROMBINE ET LEUR UTILISATION

(30) Priorität: 10.09.2001 DE 10144340; 21.09.2001 DE 10146632; 09.10.2001 DE 10149678; 21.11.2001 DE 10156995; 10.01.2002 DE 10200666
(43) Veröffentlichungstag der Anmeldung: 09.06.2004
(73) Patentinhaber: Novel Science International GmbH, 37085 Göttingen (DE)
(72) Erfinder: THÜRK, Marcel, D-37120 Bovenden (DE)
(74) Vertreter: Held, Stephan
(86) Internationale Anmeldenummer: PCT/EP2002/010137
(87) Internationale Veröffentlichungsnummer: WO 2003/022873

(56) Entgegenhaltungen:
- EP-A- 0 456 152
- EP-A- 0 498 508
- EP-A- 0 531 537
- WO-A-92/13879
- CLAESON G: "SYNTHETIC PEPTIDES AND PEPTIDOMIMETICS AS SUBSTRATES AND INHIBITORSOF THROMBIN AND OTHER PROTEASES IN THE BLOOD COAGULATION SYSTEM" BLOOD COAGULATION & FIBRINOLYSIS, RAPID COMMUNICATIONS, OXFORD,OXFORD, GB, Bd. 5, 1994, Seiten 411-436, XP000918308 ISSN: 0957-5235

## Beschreibung

Diese Erfindung betrifft biologische Wirkstoffe, die mit Thrombin interagieren und dieses hemmen. Die Wirkstoffe sind als Antikoagulationsmittel für Menschen und Tiere nützlich.

Die Erfindung betrifft auch Zusammenstellungen und Kombinationen mit diesen Stoffen für therapeutische, prophylaktische und diagnostische Zwecke.

Akute Gefäßerkrankungen, wie der myokordiale Infarkt, der Schlaganfall, die Lungenembolie, die tiefe Venenthrombose oder die periphere Gefäßverstopfung und andere Thrombosen des Blutsystems, bilden eine wesentliche gesundheitliche Gefahrenquelle. Derartige Erkrankungen werden durch vollständige oder teilweise Verstopfung des Blutgefäßes durch einen Pfropfen, der Fibrin und Blutplättchen enthält, hervorgerufen.

Gegenwärtige Methoden der Behandlung und Prophylaxe solcher Thromboseerkrankungen umfassen Therapeutika, die auf zwei verschiedenen Wegen wirken. Der erste Typ der Therapeutika verhindert die Thrombin-Aktivität oder Thrombose-Bildung und damit die Bildung des Pfropfens. Diese Medikamente verhindern auch die Entwicklung von Blutplättchen und deren Aggregation. Die zweite Kategorie von Medikamenten beschleunigt die Thrombolyse und löst den Pfropfen auf, beseitigt damit diesen aus dem Blutgefäß und hebt die Blockade des Blutflusses auf.

Heparin und Cumarin, Präparate des ersten Typs, werden in großem Maße zur Behandlung von venöser Thromboseembolien eingesetzt, in der die thrombotische Aktivität für die Entwicklung und Ausdehnung des Thrombus verantwortlich ist. Obgleich wirkungsvoll, ruft Heparin jedoch viele unerwünschte Nebenwirkungen hervor, wie Blutungen oder Thrombocytopenie. Das gleiche gilt für Cumarin, das durch die Blockade oder Verhinderung der Bildung von Prothrombin wirkt und einige Zeit bis zu seiner vollen Wirkungsentfaltung benötigt. Zusammengenommen hat dies zu einer Suche nach spezifischer wirkenden und weniger toxischen Antigerinnungsmitteln geführt, wie z.B. peptidischen Hemmern.

Hirudin ist ein natürliches vorkommendes Polypeptid, das vom Blutegel *Hirudo medicinalis* produziert wird. Dieser Wirkstoff, der in der Speicheldrüse des Blutegels synthetisiert wird, ist der wirkungsvollste bekannte natürliche Gerinnungshemmer. Hirudin ist ein direkter Thrombininhibitor und unterbindet die Koagulation des Blutes durch eine starke Bindung an das Thrombin (Kd = 2x10⁻¹⁴ M) in einem stöchiometrischen 1:1 Komplex [Stone & Hofstenge, Kinetics of the inhibition of thrombin by hirudin, Biochemistry 25, S. 4622-4628 (1986)]. Dieses wiederum verhindert, daß das Thrombin die Umwandlung von Fibrinogen zu Fibrin (dem Pfropfen) katalysiert, wie es auch alle anderen Thrombin-vermittelten Spaltungsprozesse verhindert.

In Tierstudien wurde die Effizienz von Hirudin, in gereinigter Form aus Blutegeln gewonnen, bei der Prävention von venöser Thrombose, Gefäßverstopfung und Thrombininduzierter verbreiteter intravaskulärer Koagulation demonstriert. Darüber hinaus zeigt Hirudin eine geringe Toxizität, geringe Antikörperbildung und eine schnelle Abbaubarkeit aus dem Blutkreislauf [F.Markwardt et al., Pharmacological studies on the antithrombic action of Hirudin in experimental animals, Thromb. Haemost. 47, S. 226-229 (1982)]. In Projekten, die darauf abzielen, größere Mengen von Hirudin herzustellen, wurden Versuche unternommen, das Polypeptid durch rekombinante DNA-Technologie herzustellen. Die Gegenwart eines O-sulfatisierten Tyrosin-Rests im natürlichen Hirudin und die Unfähigkeit der Mikroorganismen, eine gleichartige Modifikation durchzuführen, machten die Aussicht auf eine rekombinante Produktion biologisch aktiven Hirudins hochspekulativ. Die Beobachtung, daß desulfatisiertes Hirudin fast so wirkungsvoll ist wie das sulfierte Gegenstück [U.S.Pat.No. 4 654 302], zeigte den Weg zur Klonierung und Darstellung in verschiedenen Expressionssystemen auf, unter anderem *S. cerevisiae* [Harvey et al., Cloning and expression of cDNA coding for the anticoagulant hirudin from the bloodsucking leech, Hirudo medicinalis, PNAS 83, S. 1084-1088; Europ.Pat.Appl. 158 654, 168 342 und 171 024], *E. coli* [Bergmann et al., Chemical synthesis and expression of a gene coding for hirudin, the thrombin-specific inhibitor from the leech Hirudo medicinalis, Biol. Chem. Hoppe-Seyler 367, S. 731-740; Europ.Pat.Appl. 200 655] und auf den Spitzen eines filamentösen Phagen als Verschmelzungprotein mit Protein III (pIII) [Wirsching et al., Display of functional thrombin inhibitor hirudin on the surface of phage M13, Gene 204, S. 177-184]. Trotz dieser Fortschritte ist Hirudin in der Produktion nach wie vor recht teuer. Dennoch hat es die dritte klinische Phase durchlaufen und wurde kürzlich für die Behandlung von Heparin-induzierter Thrombocytopenia zugelassen (HMR).

Erst kürzlich wurden bei der Identifikation von Peptidfragmenten natürlichen Hirudins Erfolge erzielt, die ebenso die Koagulationszeit verlängern. Solche Peptidfragmente können jedoch aufgrund ihrer geringen Wirksamkeit bezüglich der Verhinderung der Pfropfenbildung nicht voll zufriedenstellen. So hat z.B. N-Acetyl-Hirudin₄₅₋₆₅ eine um vier Größenordnungen geringere Wirksamkeit als natürliches Hirudin, obgleich es nach wie vor ein relativ großes Molekül ist. Das Problem eher geringer Affinitäten für Thrombin wurde durch die Entwicklung von Hirulogen [U.S.Pat.No. 5 433 940] gelöst. Diese Moleküle ahmen die Wirkung von Hirudin dadurch nach, daß sie sowohl an die nach außen gerichtete Anion-bindende Seite geringer Affinität binden, wie auch an die katalytische Seite des α-Thrombins. Von daher sind Hiruloge durch eine an die Anion-bindende Thrombin-Außenseite assoziierte Hälfte, eine Verbindungsgruppe und einen gegen das katalytische Zentrum des Thrombins gerichteten Anteil charakterisiert. Das am meisten bevorzugte Hirulog ist Hirulog-8, ein Peptid aus 20 Aminosäuren, das aus dem das katalytische Zentrum hemmenden Peptid D-Phe-Pro-Arg-Pro-, einer Gly₄-Verbindungs-Sequenz und der Sequenz -Asn-Gly-Asp-Phe-Glu-Glu-Ile-Pro-Glu-Glu-Tyr-Leu-OH des Hirudins aufgebaut ist. Hirulog-8 befindet sich seit kurzem in den USA auf dem Markt.

Trotz der Fortschritte relativ hoher Wirksamkeit für Thrombin (Ki = 2,3 nM) sind Hiruloge immer noch relativ große Moleküle, die in relativ mühsamen Schemata, wie gemischten heterologen / festen Phasen, synthetisiert werden müssen. Wie Hirudin sind Hirologe nur parenteral hilfreich und müssen sorgfältig überwacht werden. Daher sind Hiruloge nicht als Leitstrukturen für kleine Moleküle geeignet, die schließlich auch oral verabreicht werden könnten.

Daher gab es einige Anstrengungen, um kleinere Peptide als potente Thrombinhemmer zu identifizieren. Bereits 1956 zeigte Bettelheim, daß das Fibrinopeptid A die Reaktion zwischen Thrombin und Fibrinogen vergleichbar hemmt. In einer gemeinsamen Forschung von Blombäck und der Nobel Pharma/Kabi in Stockholm wurden vom Fibrinopeptid A abgeleitete Peptidsequenzen mit nicht mehr als neun Aminosäuren mit guter Wirksamkeit auf Thrombin gefunden. Wesentliche Bestandteile der Wirksamkeit waren ein N-endständiges Phe und ein C-endständiges Arg, getrennt durch sieben Aminosäuren. Weniger Aminosäuren verringerten die Wirksamkeit, aber erstaunlicherweise zeigte ein Tripeptid mit N- und C-endständigem Phe bzw. Arg exzellente Wirksamkeit. Das beste Tripeptid mit hemmender Wirkung auf die Thrombin-Fibrinogen-Reaktion war Bz-Phe-Val-Arg-OMe, wobei Val wie im Fibrinopeptid ganzer Länge dem Arg vorausgeht [Blombäck et al., Synthetic peptides with anticoagulant and vaodilating activity, Scand. J. Clin. Lab. Invest. 24, S. 59-66 (1969), U.S.Pat.No. 3 826 794 (1974)]. Im Gegensatz zum Fibrinopeptid A geht Pro dem Arg in einer Reihe anderer Thrombinausschnittsbereiche, wie der des Prothrombins, des Faktors XIII und der menschlichen Wachstumshormone voraus. Auf Basis der Pro-Arg-Sequenz wurden die meisten der heute wirksamsten Thrombin-hemmenden Peptide und Peptidomimetiken entwickelt. Unter diesen wirkungsvollsten Hemmern befindet sich H-D-Phe-Pro-Arg-H (K*ᵢ* = 70 nM) [Bajuz et al., Inhibition of thrombin and trypsin by tripeptide aldehydes, Int. J. Peptide Protein Res. 12, S. 217-221 (1978); Hung.Pat. 169 870 (1974)]. Die Idee zu diesem Peptidaldehyd erwuchs aus der Entdeckung von Peptidaldehyden bakteriellen Ursprungs durch H. Umezawa. Diese so genannten Leupeptide (z.B. Ac-Leu-Leu-Arg-H) sind Hemmer des Plasmins und anderer Trypsin-ähnlicher Proteasen, jedoch nicht des Thrombins. Der Aldehyd-Kohlenstoff hat in seiner acetalen Form eine tetraedrale Struktur, die gleiche wie der Carbonyl-Kohlenstoff von Substraten in der Übergangsphase.

Aus diesen eben erwähnten Aldehyden synthetisierten Shaw et al. den irreversiblen Chlormethylketon-Hemmer H-D-Phe-Pro-Arg-CH₂-Cl mit einem Kᵢ von 25 nM [Kettner et al., H-D-Phe-Pro-Arg-CH2-Cl - a selective activity label for thrombin, Thromb. Res. 14, S.969-973 (1979)]. Entwicklungsarbeiten bei Eli Lilly führten zu N-D-Methyl-Phe-Pro-Arg-H, auch als Efegatran [tm] bekannt. Die D-Phe-Pro-Arg-Sequenz wurde kürzlich nochmals weiterentwickelt. Spekulationen, daß eine N-endständige Aminosäure mit aromatischen / lipophilen Gruppen eine größere Wirksamkeit gegenüber Thrombin ergeben könnte, führten zur Entdeckung einiger Hemmer mit neuen Aminosäuren an dieser Position, darunter das β-β-Diphenylalanin (Dpa), Phenylglyzin, Cyclohexylglyzin, Carboxy-1,2,3,4-tetrahydroisoquinolin (Tiq) [Schuman et al., Highly selective thrombin inhibitors, J. Med. Chem. 36, S.314-319 (1993)]. Die interessanteste Verbindung war D-1-Tiq-Pro-Arg-H, die verglichen mit Boc-D-Phe-Pro-Arg-H, den doppelten Wirksamkeitsgewinn ergab. Jedoch wird Trypsin in gleichem Maße wie das Thrombin gehemmt.

Aus den heute zugänglichen Daten ist klar, daß, obgleich es einige effektive antigerinnungswirksame Verbindungen gibt, ein Bedarf an leistungsfähigen Antithrombinmitteln besteht, die schnell wirken, um die Pfropfenbildung zu verhindern und die nicht mit anderen Proteaseaktivitäten, z.B. der Plasminwirkung beim Auflösen des Pfropfens interferieren.

Ausgehend von diesem Stand der Technik lag der vorliegenden Erfindung die Aufgabe zugrunde, Verbindungen bereitzustellen, die im Sinne einer Thrombinhemmung biologisch aktiv sind und die Nachteile des vorhergehend beschriebenen Standes der Technik vermeiden. Das der Erfindung zugrundeliegende Problem bestand darüberhinaus darin, Thrombin spezifisch mit geringen Wirkstoffkonzentrationen und geringer Zelltoxizität zu inhibieren.

Erfindungsgemäß wird diese Aufgabe mit den nachstehend definierten Verbindungen (I) gelöst.

Die obige Aufgabe wird durch die Verbindung mit der Formel

Y¹-(NH-X¹-C=O)-(NH-X²-C=O)-(NH-X³-C=O)-(NH-X⁴-C=O)-(NH-X⁵-C=O) (NH-X⁶-C=O)-Y² (I)

gelöst,
wobei Y¹ entweder
1. ein Wasserstoff oder
2. eine Methylgruppe oder
3. eine Acetylgruppe ist oder
4. durch ein Rückgrat aus einer Kette von 1 bis 32 Kohlenstoffatomen charakterisiert ist,
wobei (NH-X¹-C=O) ein basischer Aminosäurebaustein, vorzugsweise
1. L-Arginin oder
2. D-Arginin oder
3. L-Lysin oder
4. D-Lysin oder
5. L-Ornithin oder
6. D-Ornithin ist,
wobei (NH-X²-C=O) eine zyklische, unpolare Aminosäure, vorzugsweise
1. L-Cyclohexylalanin oder
2. D-Cyclohexylalanin oder
3. L-Cyclohexylglycin oder
4. D-Cyclohexylglycin ist,
wobei (NH-X³-C=O) eine beliebige D- oder L-Aminosäure ist, vorzugsweise
1. L-Norleucin oder
2. D-Norleucin oder
3. L-Leucin oder
4. D-Leucin oder
5. L-Isoleucin oder
6. D-ISoleucin oder
7. L-Cyclohexylalanin oder
8. D-Cyclohexylalanin oder
9. L-Cyclohexylglycin oder
10. D-Cyclohexylglycin oder
11. L-Prolin oder
12. D-Prolin oder
13. L-Asparaginsäure oder
14. D-Asparaginsäure,
15. L-Glutaminsäure oder
16. D-Glutaminsäure,
wobei (NH-X⁴-C=O) eine cyclische D- oder L-Amiosäure, vorzugsweise
1. L-Cyclohexylalanin oder
2. D-Cyclohexylalanin oder
3. L-Cyclohexylglycin oder
4. D-Cyclohexylglycin oder
5. L-Tyrosin oder
6. D-Tyrosin ist,
7. L-Phenylalanin oder
8. D-Phenylalanin ist,
wobei (NH-X⁵-C=O) eine Aminosäure mit polarer Seitenkette, vorzugsweise
1. L-Glutamin oder
2. D-Glutamin oder
3. L-Ornithin oder
4. D-Ornithin oder
5. L-Glutaminsäure oder
6. D-Glutaminsäure oder
7. L-Arginin oder
8. D-Arginin oder
9. L-Lysin oder
10. D-Lysin oder
11. L-Asparagin oder
12. D-Asparagin oder
13. L-Asparaginsäure oder
14. D-Asparaginsäure ist oder
15. durch eine chemische Bindung ersetzt wird,
wobei (NH-X⁶-C=O) eine beliebige D- oder L-Aminosäure ist, vorzugsweise
1. L-Arginin oder
2. D-Arginin oder
3. durch eine chemische Bindung ersetzt wird,
wobei Y² entweder
1. eine OH-Gruppe (die C-endständige Aminosäure hat eine endständige CarbonsäureGruppe) oder
2. eine Aminogruppe (bei der C-endständigen Aminosäure ist die Carbonsäure- durch eine Amid-Gruppe ersetzt) oder
3. ein Wasserstoff (bei der C-endständigen Aminosäure ist die Carbonsäure- durch eine Aldehyd-Gruppe ersetzt) oder
4. 7-Amido-4-methylcumarin (über die Carbonsäuregruppe kombiniert) oder
5. Paranitroanilid (über die Carbonsäuregruppe kombiniert) oder
6. durch eine Verbindungskette aus eins bis 35 Atomen ersetzt ist,
oder ein am C-Terminus und / oder am N-Terminus um nicht weniger als eine Aminosäure verkürztes Molekül und pharmazeutisch akzeptable Salze davon.

Die Erfindung betrifft auch Derivate der oben genannten Verbindungen der Formel (I).

Besonders vorteilhafte Ergebnisse werden erzielt, wenn es sich bei dem erfindungsgemäßen Peptid der Formel (I) um N-Acetyl-L-Arg-L-Cha-(NH-X³-C=O)-L-Cha-(NH-X⁵-C=O)-amid oder N-Acetyl-L-Arg-L-Cha-(NH-X³-C=O)-L-Cha-amid oder N-Acetyl-L-Arg-L-Cha-(NH-X³-C=O)-D-Tyr-(NH-X⁵-C=O)-amid handelt.

Die erfindungsgemäßen Verbindungen können zur Hemmung aller Thrombin-vermittelten oder Thrombin-assoziierten Funktionen und Prozesse verwendet werden. Pharmazeutische Zusammenstellungen, die diese Verbindungen enthalten, wie auch Methoden zur Behandlung und Prophylaxe von Gefäßerkrankungen, entzündlichen Reaktionen, Karzinomen und neurodegenerativen Erkrankungen, die diese Verbindungen verwenden, sind ebenfalls Gegenstand der Erfindung. Die Verbindungen können auch zur *ex vivo* Darstellung verwendet werden, zur Lagerung und Behandlung von Blut außerhalb des Körpers und zur Beschichtung von invasiven Geräten. Ferner können die Verbindungen einem Patienten (unter Patient wird hier ein Mensch oder Tier verstanden) in Kombination mit einem fibrinolytischem Mittel verabreicht werden, um die Wirksamkeit einer gegebenen Dosis zu erhöhen oder die zur Erzielung eines gewünschten Effekts notwendige Dosis zu verringern, wie das Auflösen und Blutpfropfens oder die Verhinderung der Wiederverstopfung des zuvor blockierten Blutgefäßes.

Aufgrund ihres hohen Potentials und der Tatsache, daß sie durch chemische Synthesetechniken hergestellt werden können, können die Verbindungen preisgünstig in kommerziell praktikablen Mengen produziert werden. Die Peptide werden in passende Salzformen wie Acetate und Sulfate umgewandelt.

Darüber hinaus sind die erfindungsgemäßen Moleküle wesentlich kleiner als Hirudin und die anderen bisher beschriebenen peptidischen Thrombinhemmer. Daher ist es unwahrscheinlicher, eine unerwünschte Reaktion des Immunsystems bei mit diesen Substanzen behandelten Patienten hervorzurufen. Dementsprechend ist der Einsatz dieser Thrombin-Hemmer nicht auf die Behandlung akuter Erkrankungen beschränkt. Diese Verbindungen können auch in der Therapie chronischer thrombo-embolitischer Erkrankungen wie der Arteriosklerose oder der Restenosis infolge einer Angioplastie, eingesetzt werden. Die erfindungsgemäßen Verbindungen können auch in einer Vielzahl anderer Anwendungen anstelle natürlichen und rekombinanten Thrombins eingesetzt werden.

Wie man aus der Offenlegung folgern kann, sind die erfindungsgemäßen Verbindungen, Zusammenstellungen und Verfahren nützlich zur Behandlung und Vorsorge verschiedener Krankheiten in Zusammenhang mit unerwünschten Wirkungen des Thrombins, wie auch für diagnostische Zwecke.

Schließlich soll erwähnt werden, daß die Moleküle dieser Erfindung als Leitstrukturen zur Entwicklung von Molekülen mit noch vorteilhafteren Eigenschaften in Hinblick auf die oben genannten Anwendungen dienen können.

Pharmazeutisch akzeptable Salze von Peptiden dieser Erfindung beinhalten die durch Säurezugabe erzeugten Salze, die aus anorganischen Säuren und Carbonsäuren gebildet werden. Die Verbindungen, die durch die Formel (I) repräsentiert werden, werden durch bekannte Methoden der Peptidkopplung hergestellt.

In einer bevorzugten Ausführungsform liegen die erfindungsgemäßen Verbindungen als Verbindungsgemisch vor, das durch seinen Gehalt an mindestens zwei der erfindungsgemäßen Verbindungen charakterisiert ist. Besonders bevorzugt ist es die mindestens zwei erfindungsgemäßen Verbindungen aus dem Verbindungen (I) auszuwählen. Bevorzugte pharmazeutisch akzeptable Salze der Verbindungen werden mit einer anorganischen Säure gebildet. Besonders bevorzugt ist dabei die Bildung eines pharmazeutisch akzeptablen Salzes mit Chlorwasserstoffsäure, Chlorsäure, Bromwasserstoffsäure, Bromsäure und / oder einer anderen Halogensäure. Eine weitere besonders bevorzugte Ausführungsform besteht in der Bildung eines pharmazeutisch akzeptablen Salzes mit Schwefelsäure und / oder Phosphorsäure. Vorteilhalft kann auch ein pharmazeutisch akzeptables Salz mit einer organischen Säure gebildet werden. Besonders bevorzugt ist dabei die Bildung des pharmazeutisch akzeptablen Salzes mit Essigsäure, Propionsäure, Malonsäure, Maleinsäure, Zitronensäure, Bernsteinsäure, Fumarsäure, Äpfelsäure, Benzoesäure, und/oder einer ähnlichen Carbonsäuren. Die durch Säurezugabe gebildeten Salze werden auf konventionelle Weise hergestellt, z.B. durch Neutralisieren der freien Basenform der Verbindungen (I) mit der Säure.

Die erfindungsgemäßen Substanzen können in Verbindungen und Methoden zur Hemmung aller Thrombin-vermittelten oder Thrombin-assoziierten Funktionen verwendet werden. Pharmazeutische Zusammensetzungen, die diese Moleküle enthalten, sind, wie auch Methoden zur Behandlung und Prophylaxe von Gefäßerkrankungen, entzündlichen Reaktionen, Karzinomen und neurodegenerativen Erkrankungen, die diese Verbindungen verwenden, ebenfalls Teil der Erfindung. Die Substanzen können auch in Zusammensetzung zur *ex vivo*-Darstellung verwendet werden, zur Lagerung und zur Behandlung von Blut außerhalb des Körpers und zur Beschichtung von invasiven Geräten. Ferner können die erfindungsgemäßen Verbindungen einem Patienten (unter Patient wird hier ein Mensch oder ein Tier verstanden) in Kombination mit einem fibrinolytischen Mittel verabreicht werden, um die Wirksamkeit einer gegebenen Dosis zu erhöhen oder um die Erzielung eines gewünschten Effektes, wie das Auflösen eines Blutpfropfens oder das Verhindern der Wiederverstopfung des zuvor blockierten Blutgefäßes notwendige Dosis zu verringern.

Aufgrund ihres hohen Potentials und der Tatsache, daß sie durch chemische Synthesetechniken hergestellt werden können, sind die erfindungsgemäßen Substanzen preisgünstig in kommerziell praktikabler Menge produzierbar. Bevorzugt werden die Peptide in passende Salzformen, wie Acetate oder Sulfate umgewandelt.

Die Erfindung betrifft auch Arzneimittel, die durch ihren Gehalt an einer oder mehreren erfindungsgemäßen Verbindungen, diese insbesondere ausgewählt aus einer der Verbindungen (I) gegebenenfalls mit den üblichen Trägerstoffen, Hilfsmitteln oder Zusatzstoffen charakterisiert ist. Ferner ist auch eine Diagnostikzusammensetzung mit einem Gehalt an einer oder mehreren, diese insbesondere ausgewählt aus einer der Verbindungen (I) der erfindungsgemäßen Verbindungen Gegenstand der Erfindung.

Ein weiterer Gegenstand der Erfindung besteht in der Verwendung der Verbindung als Thrombininhibitor sowie zur Herstellung eines Arzneimittels zur Thrombininhibierung, Inhibierung der Fibrinbildung und / oder zur Inhibierung der Bildung eines Gerinnungspfropfens.

Die Verwendung einer oder mehrerer der Verbindungen, diese insbesondere ausgewählt aus einer der Verbindungen (I) zur Herstellung einer diagnostischen Zusammensetzung ist ebenfalls Gegenstand der Erfindung.

In einer der besonders bevorzugten Ausführungsformen wird zur Herstellung der diagnostischen Zusammensetzung eine erfindungsgemäße Verbindung verwendet, bei der in der Formel (I) Y² 7-Amido-3-methylcumarin oder Paranitroanilid ist.

Die erfindungsgemäßen Verbindungen weisen gegenüber bisher bekannten Thrombininhibitoren vielfältige Vorteile auf. Insbesondere sind die Peptide leicht synthetisierbar, bereits wenig modifiziert wirksam und weisen eine hohe Wirksamkeit bei gleichzeitig hoher Spezifität und geringer Toxizität auf. Darüber hinaus dienen die kleinen Peptide, anders als Hirudin und Hirolog als Leitstrukturen für vorzugsweise oral verfügbare Wirkstoffe.

Die nachfolgenden Beispiele sollen die Erfindung näher erläutern, ohne diese einzuschränken.

Bevorzugte Ausführungsformen der vorliegenden Erfindung sind nachstehend beschrieben:
1. Eine Verbindung der Formel (I)

   Y¹-(NH-X¹-C=O)-(NH-X²-C=O)-(NH-X³-C=O)-(NH-X⁴-C=O)-(NH-X⁵-C=O)-(NH-X⁶-C=O)-Y² (I),

   wobei Y¹ entweder
   a) ein Wasserstoff oder
   b) eine Methylgruppe oder
   c) eine Acetylgruppe ist oder
   d) durch ein Rückgrat aus einer Kette von 1 bis 32 Kohlenstoffatomen charakterisiert ist,
   wobei (NH-X¹-C=O) ein basischer Aminosäurebaustein, insbesondere
   a) L-Arginin oder
   b) D-Arginin oder
   c) L-Lysin oder
   d) D-Lysin oder
   e) L-Ornithin oder
   f) D-Ornithin ist,
   wobei (NH-X²-C=O) eine cyclische, unpolare Aminosäure, insbesondere
   a) L-Cyclohexylalanin oder
   b) D-Cyclohexylalanin oder
   c) L-Cyclohexylglycin oder
   d) D-Cyclohexylglycin ist,
   wobei (NH-X³-C=O) eine beliebige D- oder L-Aminosäure, insbesondere
   a) L-Norleucin oder
   b) D-Norleucin oder
   c) L-Leucin oder
   d) D-Leucin oder
   e) L-Isoleucin oder
   f) D-Isoleucin oder
   g) L-Cyclohexylalanin oder
   h) D-Cyclohexylalanin oder
   i) L-Cyclohexylglycin oder
   j) D-Cyclohexylglycin oder
   k) L-Prolin oder
   l) D-Prolin oder
   m) L-Asparaginsäure oder
   n) D-Asparaginsäure oder
   o) L-Glutaminsäure oder
   p) D-Glutaminsäure ist,
   wobei (NH-X⁴-C=O) eine cyclische Aminosäure, insbesondere
   a) L-Cyclohexylalanin oder
   b) D-Cyclohexylalanin oder
   c) L-Cyclohexylglycin oder
   d) D-Cyclohexylglycin oder
   e) L-Tyrosin oder
   f) D-Tyrosin oder
   g) L-Phenylalanin oder
   h) D-Phenylalanin ist,
   wobei (NH-X⁵-C=O) eine Aminosäure mit polarer Seitenkette, insbesondere
   a) L-Glutamin oder
   b) D-Glutamin oder
   c) L-Ornithin oder
   d) D-Ornithin oder
   e) L-Glutaminsäure oder
   f) D-Glutaminsäure oder
   g) L-Arginin oder
   h) D-Arginin oder
   i) L-Lysin oder
   j) D-Lysin oder
   k) L-Asparagin oder
   l) D-Asparagin oder
   m) L-Asparaginsäure oder
   n) D-Asparaginsäure ist oder
   o) durch eine chemische Bindung ersetzt wird,
   wobei (NH-X⁶-C=O) eine beliebige D- oder L-Aminosäure ist, insbesondere
   a) L-Arginin oder
   b) D-Arginin oder
   c) durch eine chemische Bindung ersetzt wird,
   wobei Y² entweder
   a) eine OH-Gruppe (die C-endständige Aminosäure hat eine endständige Carbonsäure-Gruppe) oder
   b) eine Aminogruppe (bei der C-endständigen Aminosäure ist die Carbonsäuredurch eine Amid-Gruppe ersetzt) oder
   c) ein Wasserstoff (bei der C-endständigen Aminosäure ist die Carbonsäuredurch eine Aldehyd-Gruppe ersetzt) oder
   d) 7-Amido-4-methylcumarin oder (über die Carbonsäuregruppe kombiniert) oder
   e) Paranitroanilid (über die Carbonsäuregruppe kombiniert) oder
   f) durch eine Verbindungskette aus 1 bis 35 Atomen ersetzt ist,
   oder ein am C-Terminus und/oder am N-Terminus um nicht weniger als eine Aminosäure verkürztes Molekül, und pharmazeutisch akzeptable Salze davon.
2. Verbindung nach Ausführungsform 1, gekennzeichnet durch die Zusammensetzung N-Acetyl-L-Arg-L-Cha-(NH-X³-C=O)-(NH-X⁴-C=O)-(NH-X⁵C=O)(NH-X⁶-C=O)-Y² mit den angegebenen Bedeutungen und pharmazeutisch akzeptable Salze davon.
3. Verbindung nach Ausführungsform 1, gekennzeichnet durch die Zusammensetzung N-Acetyl-L-Arg-L-Cha-L-Nle-L-Cha-D-Gln-amid und pharmazeutisch akzeptable Salze davon.
4. Verbindung nach Ausführungsform 1, gekennzeichnet durch die Zusammensetzung N-Acetyl-L-Arg-L-Cha-L-Asp-L-Cha-amid und pharmazeutisch akzeptable Salze davon.
5. Verbindung nach Ausführungsform 1, gekennzeichnet durch die Zusammensetzung N-Acetyl-L-Arg-L-Cha-L-Nle-L-Cha-L-Orn-amid und pharmazeutisch akzeptable Salze davon.
6. Verbindung nach Ausführungsform 1, gekennzeichnet durch die Zusammensetzung N-Acetyl-L-Arg-L-Cha-L-Cha-L-Cha-D-Glu-amid und pharmazeutische akzeptable Salze davon.
7. Verbindung nach Ausführungsform 1, gekennzeichnet durch die Zusammensetzung N-Acetyl-L-Arg-L-Cha-D-Pro-D-Tyr-L-Arg-amid und pharmazeutisch akzeptable Salze davon.
8. Verbindung nach Ausführungsform 1, gekennzeichnet durch die Zusammensetzung N-Acetyl-L-Arg-L-Cha-L-Cha-L-Cha-L-Orn-amid und pharmazeutisch akzeptable Salze davon.
9. Verbindung nach Ausführungsform 1, gekennzeichnet durch die Zusammensetzung N-Acetyl-L-Arg-L-Cha-L-Nle-L-Cha-amid und pharmazeutisch akzeptable Salze davon.
10. Verbindung nach Ausführungsform 1, gekennzeichnet durch die Zusammensetzung N-Acetyl-L-Arg-L-Cha-L-Nle-L-Cha-D-Glu-amid und pharmazeutisch akzeptable Salze davon.
11. Verbindungsgemisch, gekennzeichnet durch einen Gehalt an mindestens zwei Verbindungen nach mindestens einem der Ausführungsformen 1 bis 10.
12. Verbindung nach einem der Ausführungsformen 1 bis 11, wobei die Verbindung als pharmazeutisch akzeptables Salz vorliegt, das mit einer anorganischen Säure gebildet wird.
13. Verbindung nach Ausführungsform 12, wobei die Verbindung als pharmazeutisch akzeptables Salz vorliegt, das mit Chlorwasserstoffsäure, Bromsäure und/oder einer anderen Halogensäure gebildet wird.
14. Verbindung nach Ausführungsform 12, wobei die Verbindung als pharmazeutisch akzeptables Salz vorliegt, das mit Schwefelsäure und/oder Phosphorsäure gebildet wird.
15. Verbindung nach mindestens einem der Ausführungsformen 1 bis 11, wobei die Verbindung als pharmazeutisch akzeptables Salz vorliegt, das mit einer organischen Säure gebildet wird.
16. Verbindung nach Ausführungsform 15, wobei die Verbindung als pharmazeutisch akzeptables Salz vorliegt, das mit Essigsäure, Propionsäure, Malonsäure, Maleinsäure, Zitronensäure, Bernsteinsäure, Malsäure, Benzoesäure, Fumarsäure und/oder einer ähnlichen Carbonsäure gebildet wird.
17. Arzneimittel, gekennzeichnet durch seinen Gehalt an einer oder mehreren Verbindungen nach mindestens einem der Ausführungsformen 1 bis 16 neben üblichen Trägerstoffen, Hilfsmitteln und/oder Zusatzstoffen.
18. Diagnostische Zusammensetzung, gekennzeichnet durch ihren Gehalt an einer oder mehreren Verbindungen nach mindestens einem der Ausführungsformen 1 bis 16.
19. Verwendung einer oder mehrerer Verbindungen nach mindestens einem der Ausführungsformen 1 bis 16 zur Herstellung eines Arzneimittels zur Thrombinhemmung, Inhibierung der Fibrinbildung und/oder zur Inhibierung der Bildung eines Gerinnungspfropfens.
20. Verwendung einer oder mehrerer Verbindungen nach mindestens einem der Ausführungsformen 1 bis 16 zur Herstellung einer diagnostischen Zusammensetzung.
21. Verwendung nach Ausführungsform 20, dadurch gekennzeichnet, dass in der Verbindung der Formel (I) Y² 7-Amido-4-methylcumarin ist.
22. Verwendung nach Ausführungsform 20, dadurch gekennzeichnet, dass in der Verbindung der Formel (I) Y² Paranitroanilid ist.
23. Eine Methode zur Thrombinhemmung von Mensch und Tier, die eine wirkungsvolle Menge einer Verbindung aus Ausführungsform 1 beinhaltet.
24. Eine pharmazeutische Zusammenstellung, die eine wirkungsvolle Pfropfenverhindernde Menge einer Verbindung aus Ausführungsform 1 und einem pharmazeutisch akzeptablen Träger umfasst.
25. Eine Diagnosemethode, die eine Verbindung des Ausführungsforms 1 zur ThrombinHemmung bei Menschen und Säugetieren verwendet.

### Beispiel 1

### N-Acetyl-L-Arg-L-cha-L-Nle-L-Cha-D-Gln-amid

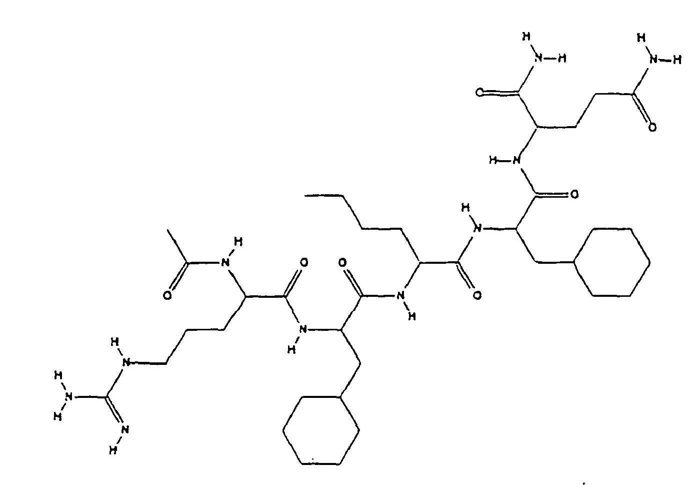

Dieses Peptid wurde durch eine Festphasen-Synthese mithilfe eines ABIMED-Synthesizers AMS 96 (ABIMED Analysen-Technik GmbH, Langenfeld, Deutschland) hergestellt. Im Detail ließ man 1 meq Rink Amid-Harz sequentiell mit 2x5 meq geschützter Aminosäure reagieren. Die Aktivierung erfolgte mit 2x5 meq TBTU (O-benzotriazol-1-yl)-N,N,N',N'-tetramethyluroniumtetrafluoroborat. Nach bis zu 6 Zyklen der Synthese wurde der N-Terminus mit Essigsäureanhydrid acetyliert. Dann wurde der Schutz des Peptids durch eine Behandlung mit 90% TFA, 2,5% Triisopropylsilan, 2,5% H₂O und 5% Dichlormethan aufgehoben. Im selben Schritt erfolgte die Entkopplung des Peptids von seinem Träger. Die Testverbindung wurde anschließend an einen Trocknungsschritt in 20 µl Trifluoressigsäure angelöst, und dann mit 2x750 µl kaltem Butylether bei - 20°Celsius inkubiert. Nach Zentrifugation wurde der Überstand abgenommen und der Rest Ether verdunstet. Die Identität der Produkte wurde stichprobenartig durch Massenspektroskopie bestätigt.

Die Hemmung des Thrombins wurde durch in-vitro-Hemmung der Amidase-Aktivität des Thrombins bestimmt und beträgt bei einer Peptidkonzentration von 25 µM 66% . Die Werte der hemmenden Konstante Kᵢ wurden aus Assays gewonnen, in denen Thrombin das fluorogene Substrat Tos-Gly-Pro-Arg-(7-amino-4-Methylcumarin) hydrolisiert. Die Assays wurde in 30 µl Assaypuffer (0,05 M Tris, 0,1 M NaCl, 0,1% PEG 8000, pH 7,6) mit 10 µl humaner Thrombinlösung (10⁻⁵ U/µl im Assaypuffer) und 140 µl einer Lösung des fluorogenen Substrats in einem Assaypuffer bei einer Konzentration v on 30 µM durchgeführt. Lösungen der Testverbindung (10µl) wurden bei verschiedenen Konzentrationen hinzugefügt. Die Raten der Hydrolyse des Substrats wurden durch Überwachung der Reaktionen bei 460 nm der Freisetzung von 7-Amino-4-Methylcumarin unter Verwendung von AMC gemessen. Die Reaktion erreichte einen Gleichgewichtszustand innerhalb von drei Minuten, nachdem Thrombin mit dem Substrat und einem Hemmer vermischt wurden. Die kinetischen Daten der konkurrierenden Hemmung (Kₘ, Vₘₐₓ und Kᵢ) wurden mithilfe der Hanesdarstellung analysiert (A/V gegen A bei verschiedenen Werten von i).

### Beispiel 2

### N-Acetyl-L-Arg-L-Cha-L-Asp-L-Cha-amid

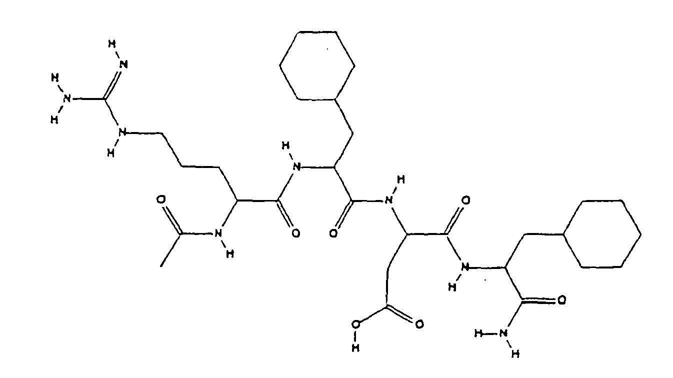

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 25 µM 68%.

### Beispiel 3

### N-Acetyl-L-Arg-L-Cha-L-Nle-L-Cha-L-Orn-amid

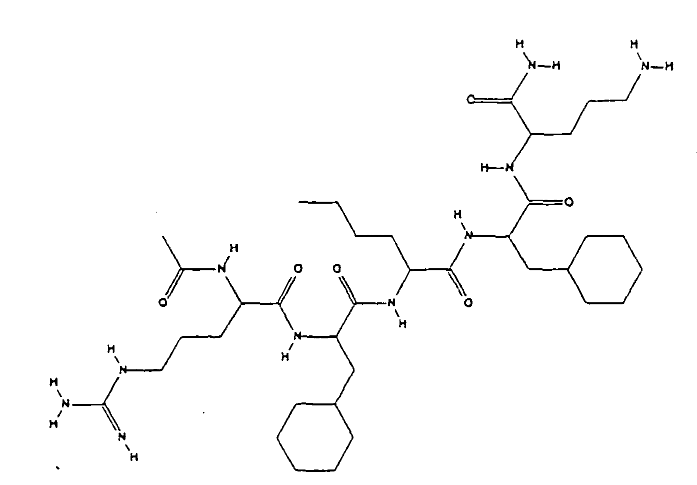

Dieses Peptid wurde wie in Beispiel 1 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 10 µM 63% .

### Beispiel 4

### N-Acetyl-L-Arg-L-Cha-L-Cha-L-Cha-D-Glu-amid

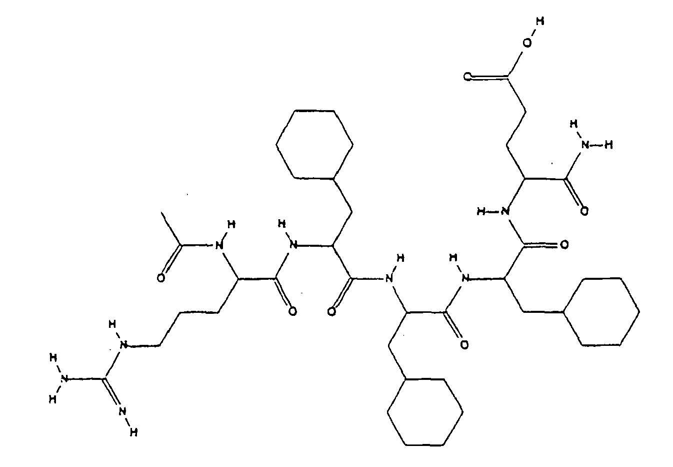

Dieses Peptid wurde wie in Beispiel 1 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 10 µM 78% .

### Beispiel 5

### N-Acetyl-L-Arg-L-Cha-D-Pro-D-Tyr-L-Arg-amid

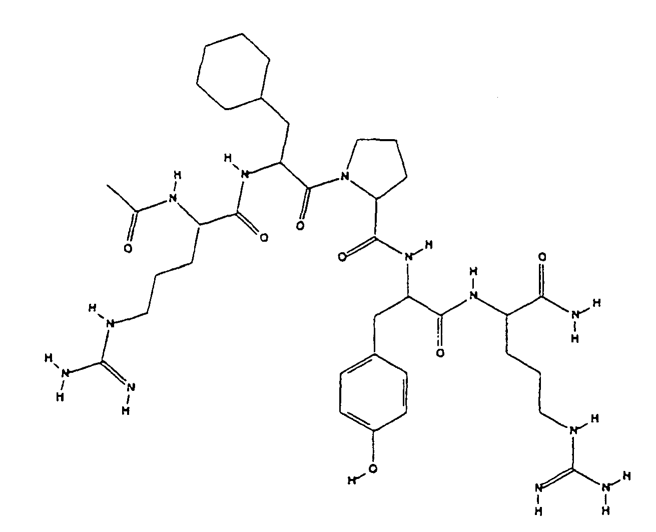

Dieses Peptid wurde wie in Beispiel 1 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 10 µM 98%.

### Beispiel 6

### N-Acetyl-L-Arg-L-Cha-L-Cha-L-Cha-L-Orn-amid

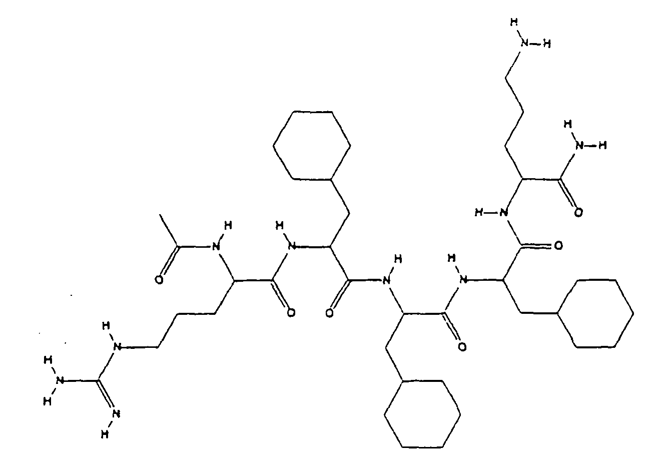

Dieses Peptid wurde wie in Beispiel 1 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 10 µM 71% .

### Beispiel 7

### N-Acetyl-L-Arg-L-Cha-L-Nle-L-Cha-amid

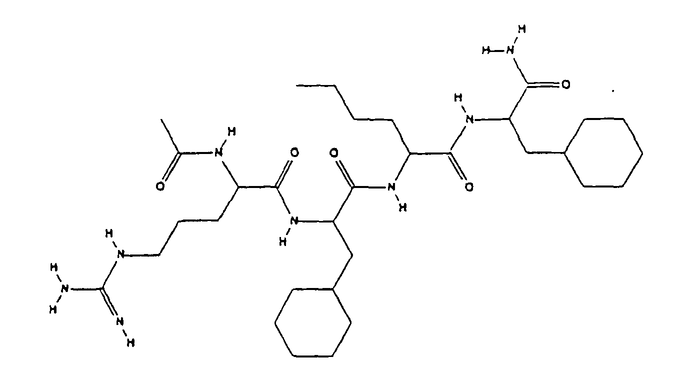

Dieses Peptid wurde wie in Beispiel 1 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 1 bestimmt und beträgt bei einer Peptidkonzentration von 10 µM 60%.

### Beispiel 8

### N-Acetyl-L-Arg-L-Cha-L-Nle-L-Cha-D-Glu-amid

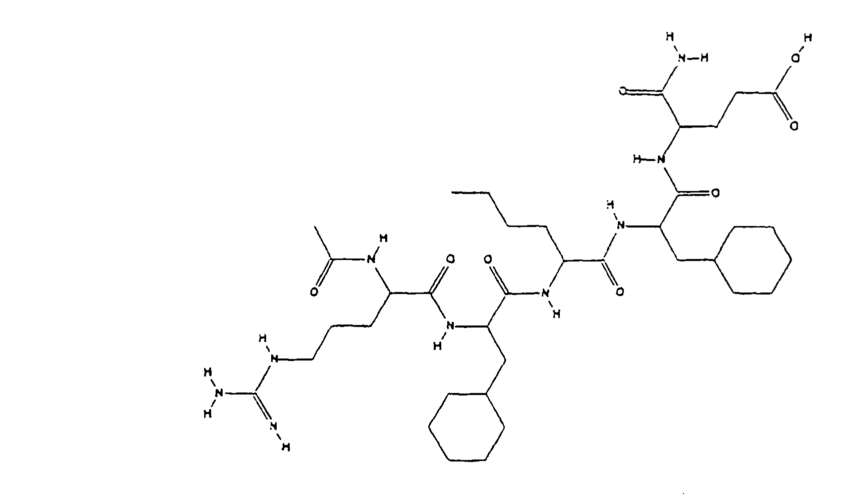

Dieses Peptid wurde wie in Beispiel 1 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 1 bestimmt und beträgt bei einer Peptidkonzentration von 1 µM 35%.

### Beispiel 9 (nicht erfindungsgemäß)

### N-Acetyl-D-Val-L-Ala-L-Cha-D-Pro-D-Tyr-L-Arg-amid

Dieses Peptid wurde durch eine Festphasen-Synthese mithilfe eines ABIMED-Synthesizers AMS 96 (ABIMED Analysen-Technik GmbH, Langenfeld, Deutschland) hergestellt. Im Detail ließ man 1 meq Rink Amid-Harz sequentiell mit 2 x 5 meq geschützter Aminosäure reagieren. Die Aktivierung erfolgte mit 2x5 meq TBTU (O-benzotriazol-1-yl)-N,N,N',N'-tetramethyluroniumtetrafluoroborat. Nach bis zu 6 Zyklen der Synthese wurde der N-Terminus mit Essigsäureanhydrid acetyliert. Dann wurden der Schutz des Peptids durch eine Behandlung mit 90% TFA, 2,5% Triisopropylsilan, 2,5% H₂O und 5% Dichlormethan aufgehoben. Im selben Schritt erfolgte die Entkopplung des Peptids von seinem Träger. Die Testverbindung wurde anschließend an einen Trocknungsschritt in 20 µl Trifluoressigsäure angelöst, und dann mit 2x750 µl kaltem Butylether bei - 20 °Celsius inkubiert. Nach Zentrifugation wurde der Überstand abgenommen und der Rest Ether verdunstet: Die Identität der Produkte wurde stichprobenartig durch Massenspektroskopie bestätigt.

Die Hemmung des Thrombins wurde durch in-vitro-Hemmung der Amidase-Aktivität des Thrombins bestimmt und beträgt bei einer Peptidkonzentration von 1 µM 53%. Die Werte der hemmenden Konstante Kᵢ wurden aus Assays gewonnen, in denen Thrombin das fluorogene Substrat Tos-Gly-Pro-Arg-(7-amino-4-Methylcumarin) hydrolisiert. Die Assays wurde in 30 µl Assaypuffer (0,05 M Tris, 0,1 M NaCl, 0,1% PEG 8000, pH 7,6) mit 10 µl humaner Thrombinlösung (10⁻⁵ U/µl im Assaypuffer) und 140 µl einer Lösung des fluorogenen Substrats in einem Assaypuffer bei einer Konzentration von 30 µM durchgeführt. Lösungen der Testverbindung (10 µl) wurden bei verschiedenen Konzentrationen hinzugefügt. Die Raten der Hydrolyse des Substrats wurden durch Überwachung der Reaktionen bei 460 nm der Freisetzung von 7-Amino-4-Methylcumarin unter Verwendung von AMC gemessen. Die Reaktion erreichte einen Gleichgewichtszustand innerhalb von drei Minuten, nachdem Thrombin mit dem Substrat und einem Hemmer vermischt wurden. Die kinetischen Daten der konkurrierenden Hemmung (Kₘ, Vₘₐₓ und Kᵢ) wurden mittels der Darstellung nach Hanes analysiert (A/V gegen A bei verschiedenen Werten von A, dabei ist A die Substratkonzentration und V die Reaktionsgeschwindigkeit).

### Beispiel 10 (nicht erfindungsgemäß)

### N-Acetyl-L-Asp-L-Ser-L-Cha-D-Pro-D-Tyr-L-Arg-amid

Dieses Peptid wurde wie in Beispiel 9 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 9 bestimmt und beträgt bei einer Peptidkonzentration von 1 µM 51%.

### Bespiel 11 (nicht erfindungsgemäß)

### N-Acetyl-L-Ile-L-Cha-D-Pro-D-Tyr-L-Arg-amid

Dieses Peptid wurde wie in Beispiel 9 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 9 bestimmt und beträgt bei einer Peptidkonzentration von 1 µM 48% .

### Beispiel 12 (nicht erfindungsgemäß)

### N-Acetyl-D-Val-L-Cha-D-Pro-D-Tyr-L-Arg-amid

Dieses Peptid wurde wie in Beispiel 9 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 9 bestimmt und beträgt bei einer Peptidkonzentration von 1 µm 46%.

### Beispiel 13 (nicht erfindungsgemäß)

### N-Acetyl-L-Ser-L-Ser-L-Cha-D-Pro-D-Tyr-L-Arg-amid

Dieses Peptid wurde wie in Beispiel 9 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 9 bestimmt und beträgt bei einer Peptidkonzentration von 1 µM 49%.

### Beispiel 14 (nicht erfindungsgemäß)

### N-Acetyl-D-Lys-D-Pro-L-Cha-D-Pro-D-Tyr-L-Arg-amid

Dieses Peptid wurde wie in Beispiel 9 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 9 bestimmt und beträgt bei einer Peptidkonzentration von 1 µM 53%.

### Beispiel 15 (nicht erfindungsgemäß)

### N-Acetyl-L-Tyr-L-Cit-L-Cha-D-Pro-D-Tyr-L-Arg-amid

Dieses Peptid wurde wie in Beispiel 9 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 9 bestimmt und beträgt bei einer Peptidkonzentration von 1 µM 51%.

### Beispiel 16 (nicht erfindungsgemäß)

### N-Acetyl-L-Ser-D-Val-L-Cha-D-Pro-D-Tyr-L-Arg-amid

Dieses Peptid wurde wie in Beispiel 9 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 9 bestimmt und beträgt bei einer Peptidkonzentration von 1 µM 48%.

### Beispiel 17 (nicht erfindungsgemäß)

### N-Acetyl-L-Ser-L-Ala-L-Cha-D-Pro-D-Tyr-L-Arg-amid

Dieses Peptid wurde wie in Beispiel 9 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 9 bestimmt und beträgt bei einer Peptidkonzentration von 1 µM 48% .

### Beispiel 18 (nicht erfindungsgemäß)

### N-Acetyl-L-Ser-L-Arg-L-Cha-D-Pro-D-Tyr-L-Arg-amid

Dieses Peptid wurde wie in Beispiel 9 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 9 bestimmt und beträgt bei einer Peptidkonzentration von 1 µM 67%.

### Beispiel 19 (nicht erfindungsgemäß)

### N-Acetyl-L-Ser-L-Cha-L-Cha-D-Pro-D-Tyr-L-Arg-amid

Dieses Peptid wurde wie in Beispiel 9 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 9 bestimmt und beträgt bei einer Peptidkonzentration von 1 µM 43%.

### Beispiel 20 (nicht erfindungsgemäß)

### N-Acetyl-D-Lys-L-Nle-L-Cha-D-Pro-D-Tyr-L-Arg-amid

Dieses Peptid wurde wie in Beispiel 9 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 9 bestimmt und beträgt bei einer Peptidkonzentration von 1 µM 44%.

### Beispiel 21

### N-Acetyl-L-Arg-L-Cha-D-Pro-D-Tyr-L-Arg-amid

Dieses Peptid wurde wie in Beispiel 9 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 9 bestimmt und beträgt bei einer Peptidkonzentration von 1 µM 38%.

### Beispiel 22 (nicht erfindungsgemäß)

### N-Acetyl-L-Ser-L-Cha-D-Pro-D-Tyr-L-Arg-amid

Dieses Peptid wurde wie in Beispiel 9 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 9 bestimmt und beträgt bei einer Peptidkonzentration von 1 µM 31%.

### Beispiel 23 (nicht erfindungsgemäß)

### N-Acetyl-L-Ser-D-Glu-L-Cha-D-Pro-D-Tyr-L-Arg-amid

Dieses Peptid wurde wie in Beispiel 9 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 9 bestimmt und beträgt bei einer Peptidkonzentration von 1 µM 21%.

### Beispiel 24 (nicht erfindungsgemäß)

### N-Acetyl-D-Tyr-L-Cha-D-Pro-D-Tyr-L-Arg-amid

Dieses Peptid wurde wie in Beispiel 9 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 9 bestimmt und beträgt bei einer Peptidkonzentration von 1 µM 27%.

### Beispiel 25

### N-Acetyl-D-Gln-D-His-L-Cha-D-Pro-D-Tyr-L-Arg-amid (nicht erfindungsgemäß)

Dieses Peptid wurde durch eine Festphasen-Synthese mithilfe eines ABIMED-Synthesizers AMS 96 (ABIMED Analysen-Technik GmbH, Langenfeld, Deutschland) hergestellt. Im Detail ließ man 1 meq Rink Amid-Harz sequentiell mit 2 x 5 meq geschützter Aminosäure reagieren. Die Aktivierung erfolgte mit 2x5 meq TBTU (O-benzotriazol-1-yl)-N, N, N' ,N'-tetramethyluroniumtetrafluoroborat. Nach bis zu 6 Zyklen der Synthese wurde der N-Terminus mit Essigsäureanhydrid acetyliert. Dann wurde der Schutz des Peptids durch eine Behandlung mit 90% TFA, 2,5% Triisopropylsilan, 2,5% H₂O und 5% Dichlormethan aufgehoben. Im selben Schritt erfolgte die Entkopplung des Peptids von seinem Träger. Die Testverbindung wurde anschließend an einen Trocknungsschritt in 20 µl Trifluoressigsäure angelöst, und dann mit 2x750 µl kaltem Butylether bei - 20 °Celsius inkubiert. Nach Zentrifugation wurde der Überstand abgenommen und der Rest Ether verdunstet. Die Identität der Produkte wurde stichprobenartig durch Massenspektroskopie bestätigt.

Die Hemmung des Thrombins wurde durch in-vitro-Hemmung der Amidase-Aktivität des Thrombins bestimmt und beträgt bei einer Peptidkonzentration von 1 µM 59%. Die Werte der hemmenden Konstante Kᵢ wurden aus Assays gewonnen, in denen Thrombin das fluorogene Substrat Tos-Gly-Pro-Arg-(7-amino-4-Methylcumarin) hydrolisiert. Die Assays wurde in 30 µl Assaypuffer (0,05 M Tris, 0,1 M NaCl, 0,1% PEG 8000, pH 7,6) mit 10 µl humaner Thrombinlösung (10⁻⁵ U/µl im Assaypuffer) und 140 µl einer Lösung des fluorogenen Substrats in einem Assaypuffer bei einer Konzentration von 30 µM durchgeführt. Lösungen der Testverbindung (10 µl) wurden bei verschiedenen Konzentrationen hinzugefügt. Die Raten der Hydrolyse des Substrats wurden durch Überwachung der Reaktionen bei 460 nm der Freisetzung von 7-Amino-4-Methylcumarin unter Verwendung von AMC gemessen. Die Reaktion erreichte einen Gleichgewichtszustand innerhalb von drei Minuten, nachdem Thrombin mit dem Substrat und einem Hemmer vermischt wurden. Die kinetischen Daten der konkurrierenden Hemmung (Kₘ, Vₘₐₓ und Kᵢ) wurden mittels der Darstellung nach Hanes analysiert (A/V gegen A bei verschiedenen Werten von A, dabei ist A die Substratkonzentration und V die Reaktionsgeschwindigkeit).

### Beispiel 26 (nicht erfindungsgemäß)

### N-Acetyl-D-Glu-L-Cha-D-Pro-D-Tyr-L-Arg-amid

Dieses Peptid wurde wie in Beispiel 25 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 25 bestimmt und beträgt bei einer Peptidkonzentration von 1 µM 58%.

### Beispiel 27 (nicht erfindungsgemäß)

### N-Acetyl-D-Val-D-His-L-Cha-D-Pro-D-Tyr-L-Arg-amid

Dieses Peptid wurde wie in Beispiel 25 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 25 bestimmt und beträgt bei einer Peptidkonzentration von 1 µM 51%.

### Beispiel 28 (nicht erfindungsgemäß)

### N-Acetyl-L-Ala-L-Cha-D-Pro-D-Tyr-L-Arg-amid

Dieses Peptid wurde wie in Beispiel 25 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 25 bestimmt und beträgt bei einer Peptidkonzentration von 1 µM 44%.

### Beispiel 29 (nicht erfindungsgemäß)

### N-Acetyl-L-Ile-L-Arg-L-Cha-D-Pro-D-Tyr-L-Arg-amid

Dieses Peptid wurde wie in Beispiel 25 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 25 bestimmt und beträgt bei einer Peptidkonzentration von 1 µM 61%.

### Beispiel 30 (nicht erfindungsgemäß)

### N-Acetyl-L-Tyr-L-Cit-L-Cha-D-Pro-D-Tyr-L-Arg-amid

Dieses Peptid wurde wie in Beispiel 25 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 25 bestimmt und beträgt bei einer Peptidkonzentration von 1 µM 55% .

### Beispiel 31 (nicht erfindungsgemäß)

### N-Acetyl-L-Ser-L-Ser-L-Cha-D-Pro-D-Tyr-L-Arg-amid

Dieses Peptid wurde wie in Beispiel 25 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 25 bestimmt und beträgt bei einer Peptidkonzentration von 1 µM 40%.

### Beispiel 32 (nicht erfindungsgemäß)

### N-Acetyl-D-Val-L-Cha-D-Pro-D-Tyr-L-Arg-amid

Dieses Peptid wurde wie in Beispiel 25 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 25 bestimmt und beträgt bei einer Peptidkonzentration von 1 µM 45%.

### Beispiel 33 (nicht erfindungsgemäß)

### N-Acetyl-L-Trp-L-Cha-D-Pro-D-Tyr-L-Arg-amid

Dieses Peptid wurde wie in Beispiel 25 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 25 bestimmt und beträgt bei einer Peptidkonzentration von 1 µM 63% .

### Beispiel 34 (nicht erfindungsgemäß)

### N-Acetyl-L-Ser-L-Ala-L-Cha-D-Pro-D-Tyr-L-Arg-amid

Dieses Peptid wurde wie in Beispiel 25 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 25 bestimmt und beträgt bei einer Peptidkonzentration von 1 µM 47%.

### Beispiel 35 (nicht erfindungsgemäß)

### N-Acetyl-L-Ser-L-Arg-L-Cha-D-Pro-D-Tyr-L-Arg-amid

Dieses Peptid wurde wie in Beispiel 25 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 25 bestimmt und beträgt bei einer Peptidkonzentration von 1 µM 51%.

### Beispiel 36 (nicht erfindungsgemäß)

### N-Acetyl-D-Lys-L-Nle-L-Cha-D-Pro-D-Tyr-L-Arg-amid

Dieses Peptid wurde wie in Beispiel 25 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 25 bestimmt und beträgt bei einer Peptidkonzentration von 1 µM 48% .

### Beispiel 37 (nicht erfindungsgemäß)

### N-Acetyl-D-Tyr-L-Cha-D-Pro-D-Tyr-L-Arg-amid

Dieses Peptid wurde wie in Beispiel 25 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 25 bestimmt und beträgt bei einer Peptidkonzentration von 1 µM 47%.

### Beispiel 38

### N-Acetyl-L-Arg-L-Cha-D-Pro-D-Tyr-L-Arg-amid

Dieses Peptid wurde wie in Beispiel 25 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 25 bestimmt und beträgt bei einer Peptidkonzentration von 1 µM 47%.

### Beispiel 39 (nicht erfindungsgemäß)

### N-Acetyl-L-Tyr-D-Pro-L-Cha-D-Pro-D-Tyr-L-Arg-amid

Dieses Peptid wurde wie in Beispiel 25 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 25 bestimmt und beträgt bei einer Peptidkonzentration von 1 µM 44% .

### Beispiel 40 (nicht erfindungsgemäß)

### N-Acetyl-D-Gln-D-His-L-Cha-D-Pro-D-Tyr-L-Arg-amid

Dieses Peptid wurde durch eine Festphasen-Synthese mithilfe eines ABIMED-Synthesizers AMS 96 (ABIMED Analysen-Technik GmbH, Langenfeld, Deutschland) hergestellt. Im Detail ließ man 1 meq Rink Amid-Harz sequentiell mit 2 x 5 meq geschützter Aminosäure reagieren. Die Aktivierung erfolgte mit 2 x 5 meq TBTU (O-benzotriazol-1-yl)-N, N, N' ,N' -tetramethyluroniumtetrafluoroborat. Nach bis zu 6 Zyklen der Synthese wurde der N-Terminus mit Essigsäureanhydrid acetyliert. Dann wurde der Schutz des Peptids durch eine Behandlung mit 90% TFA, 2,5% Triisopropylsilan, 2,5% H₂O und 5% Dichlormethan aufgehoben. Im selben Schritt erfolgte die Entkopplung des Peptids von seinem Träger. Die Testverbindung wurde anschließend an einen Trocknungsschritt in 20 µl Trifluoressigsäure angelöst, und dann mit 2x750 µl kaltem Butylether bei - 20°Celsius inkubiert. Nach Zentrifugation wurde der Überstand abgenommen und der Rest Ether verdunstet. Die Identität der Produkte wurde stichprobenartig durch Massenspektroskopie bestätigt.

Die Hemmung des Thrombins wurde durch in-vitro-Hemmung der Amidase-Aktivität des Thrombins bestimmt und beträgt bei einer Peptidkonzentration von 1 µM 59% . Die Werte der hemmenden Konstante Kᵢ wurden aus Assays gewonnen, in denen Thrombin das fluorogene Substrat Tos-Gly-Pro-Arg-(7-amino-4-Methylcumarin) hydrolisiert. Die Assays wurde in 30 µl Assaypuffer (0,05 M Tris, 0,1 M NaCl, 0,1% PEG 8000, pH 7,6) mit 10 µl humaner Thrombinlösung (10⁻⁵ U/µl im Assaypuffer) und 140 µl einer Lösung des fluorogenen Substrats in einem Assaypuffer bei einer Konzentration von 30 µM durchgeführt. Lösungen der Testverbindung (10 µl) wurden bei verschiedenen Konzentrationen hinzugefügt. Die Raten der Hydrolyse des Substrats wurden durch Überwachung der Reaktionen bei 460 nm der Freisetzung von 7-Amino-4-Methylcumarin unter Verwendung von AMC gemessen. Die Reaktion erreichte einen Gleichgewichtszustand innerhalb von drei Minuten, nachdem Thrombin mit dem Substrat und einem Hemmer vermischt wurden. Die kinetischen Daten der konkurrierenden Hemmung (Kₘ, Vₘₐₓ und Ki) wurden mittels der Darstellung nach Hanes analysiert (A/V gegen A bei verschiedenen Werten von A, dabei ist A die Substratkonzentration und V die Reaktionsgeschwindigkeit).

### Beispiel 41 (nicht erfindungsgemäß)

### N-Acetyl-D-Glu-L-Cha-D-Pro-D-Tyr-L-Arg-amid

Dieses Peptid wurde wie in Beispiel 40 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 40 bestimmt und beträgt bei einer Peptidkonzentration von 1 µM 58%.

### Beispiel 42 (nicht erfindungsgemäß)

### N-Acetyl-D-Val-D-His-L-Cha-D-Pro-D-Tyr-L-Arg-amid

Dieses Peptid wurde wie in Beispiel 40 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 40 bestimmt und beträgt bei einer Peptid-konzentration von 1 µM 51%.

### Beispiel 43 (nicht erfindungsgemäß)

### N-Acetyl-L-Ala-L-Cha-D-Pro-D-Tyr-L-Arg-amid

Dieses Peptid wurde wie in Beispiel 40 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 40 bestimmt und beträgt bei einer Peptidkonzentration von 1 µM 44%.

### Beispiel 44 (nicht erfindungsgemäß)

### N-Acetyl-L-Ile-L-Arg-L-Cha-D-Pro-D-Tyr-L-Arg-amid

Dieses Peptid wurde wie in Beispiel 40 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 40 bestimmt und beträgt bei einer Peptidkonzentration von 1 µM 61% .

### Beispiel 45 (nicht erfindungsgemäß)

### N-Acetyl-L-Tyr-L-Cit-L-Cha-D-Pro-D-Tyr-L-Arg-amid

Dieses Peptid wurde wie in Beispiel 40 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 40 bestimmt und beträgt bei einer Peptidkonzentration von 1 µM 55% .

### Beispiel 46 (nicht erfindungsgemäß)

### N-Acetyl-L-Ser-L-Ser-L-Cha-D-Pro-D-Tyr-L-Arg-amid

Dieses Peptid wurde wie in Beispiel 40 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 40 bestimmt und beträgt bei einer Peptidkonzentration von 1 µM 40%.

### Beispiel 47 (nicht erfindungsgemäß)

### N-Acetyl-D-Val-L-Cha-D-Pro-D-Tyr-L-Arg-amid

Dieses Peptid wurde wie in Beispiel 40 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 40 bestimmt und beträgt bei einer Peptidkonzentration von 1 µM 45%.

### Beispiel 48 (nicht erfindungsgemäß)

### N-Acetyl-L-Trp-L-Cha-D-Pro-D-Tyr-L-Arg-amid

Dieses Peptid wurde wie in Beispiel 40 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 40 bestimmt und beträgt bei einer Peptidkonzentration von 1 µM 63%.

### Beispiel 49 (nicht erfindungsgemäß)

### N-Acetyl-L-Ser-L-Ala-L-Cha-D-Pro-D-Tyr-L-Arg-amid

Dieses Peptid wurde wie in Beispiel 40 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 40 bestimmt und beträgt bei einer Peptidkonzentration von 1 µM 47%.

### Beispiel 50 (nicht erfindungsgemäß)

### N-Acetyl-L-Ser-L-Arg-L-Cha-D-Pro-D-Tyr-L-Arg-amid

Dieses Peptid wurde wie in Beispiel 40 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 40 bestimmt und beträgt bei einer Peptidkonzentration von 1 µM 51%.

### Beispiel 51 (nicht erfindungsgemäß)

### N-Acetyl-D-Lys-L-Nle-L-Cha-D-Pro-D-Tyr-L-Arg-amid

Dieses Peptid wurde wie in Beispiel 40 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 40 bestimmt und beträgt bei einer Peptidkonzentration von 1 µM 48% .

### Beispiel 52 (nicht erfindungsgemäß)

### N-Acetyl-D-Tyr-L-Cha-D-Pro-D-Tyr-L-Arg-amid

Dieses Peptid wurde wie in Beispiel 40 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 40 bestimmt und beträgt bei einer Peptidkonzentration von 1 µM 47%.

### Beispiel 53

### N-Acetyl-L-Arg-L-Cha-D-Pro-D-Tyr-L-Arg-amid

Dieses Peptid wurde wie in Beispiel 40 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 40 bestimmt und beträgt bei einer Peptidkonzentration von 1 µM 47%.

### Beispiel 54 (nicht erfindungsgemäß)

### N-Acetyl-L-Tyr-D-Pro-L-Cha-D-Pro-D-Tyr-L-Arg-amid

Dieses Peptid wurde wie in Beispiel 40 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 40 bestimmt und beträgt bei einer Peptidkonzentration von 1 µM 44%.

### Beispiel 55 (nicht erfindungsgemäß)

### N-Acetyl-L-Ala-D-Cha-L-Aze-D-Tyr-L-Arg-amid

Dieses Peptid wurde wie in Beispiel 40 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 40 bestimmt und beträgt bei einer Peptidkonzentration von 1 µM 63%.

### Beispiel 56 (nicht erfindungsgemäß)

### N-Acetyl-L-Ala-D-Cha-L-Pro-D-Tyr-L-Har-amid

Dieses Peptid wurde wie in Beispiel 40 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 40 bestimmt und beträgt bei einer Peptidkonzentration von 1 µM 56%.

### Beispiel 57 (nicht erfindungsgemäß)

### N-Acetyl-L-Ala-D-Cha-L-Aze-D-Tyr-L-Har-amid

Dieses Peptid wurde wie in Beispiel 40 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 40 bestimmt und beträgt bei einer Peptidkonzentration von 1 µM 83%.

### Beispiel 58 (nicht erfindungsgemäß)

### N-Acetyl-D-GLn-D-His-L-Cha-D-Pro-D-Tyr-L-Arg-amid

Dieses Peptid wurde durch eine Festphasen-Synthese mithilfe eines ABIMED-Synthesizers AMS 96 (ABIMED Analysen-Technik GmbH, Langenfeld, Deutschland) hergestellt. Im Detail ließ man 1 meq Rink Amid-Harz sequentiell mit 2 x 5 meq geschützter Aminosäure reagieren. Die Aktivierung erfolgte mit 2 x 5 meq TBTU (O-benzotriazol-1-yl)-N, N, N' ,N' -tetramethyluroniumtetrafluoroborat. Nach bis zu 6 Zyklen der Synthese wurde der N-Terminus mit Essigsäureanhydrid acetyliert. Dann wurde der Schutz des Peptids durch eine Behandlung mit 90% TFA, 2,5% Triisopropylsilan, 2,5% H₂O und 5% Dichlormethan aufgehoben. Im selben Schritt erfolgte die Entkopplung des Peptids von seinem Träger. Die Testverbindung wurde anschließend an einen Trocknungsschritt in 20 µl Trifluoressigsäure angelöst, und dann mit 2x750 µl kaltem Butylether bei - 20 °Celsius inkubiert. Nach Zentrifugation wurde der Überstand abgenommen und der Rest Ether verdunstet. Die Identität der Produkte wurde stichprobenartig durch Massenspektroskopie bestätigt.

Die Hemmung des Thrombins wurde durch in-vitro-Hemmung der Amidase-Aktivität des Thrombins bestimmt und beträgt bei einer Peptidkonzentration von 1 µM 59%. Die Werte der hemmenden Konstante Kᵢ wurden aus Assays gewonnen, in denen Thrombin das fluorogene Substrat Tos-Gly-Pro-Arg-(7-amino-4-Methylcumarin) hydrolisiert. Die Assays wurde in 30 µl Assaypuffer (0,05 M Tris, 0,1 M NaCl, 0,1% PEG 8000, pH 7,6) mit 10 µl humaner Thrombinlösung (10⁻⁵ U/µl im Assaypuffer) und 140 µl einer Lösung des fluorogenen Substrats in einem Assaypuffer bei einer Konzentration von 30 µM durchgeführt. Lösungen der Testverbindung (10 µl) wurden bei verschiedenen Konzentrationen hinzugefügt. Die Raten der Hydrolyse des Substrats wurden durch Überwachung der Reaktionen bei 460 nm der Freisetzung von 7-Amino-4-Methylcumarin unter Verwendung von AMC gemessen. Die Reaktion erreichte einen Gleichgewichtszustand innerhalb von drei Minuten, nachdem Thrombin mit dem Substrat und-einem Hemmer vermischt wurden. Die kinetischen Daten der konkurrierenden Hemmung (Kₘ, Vₘₐₓ und Ki) wurden mittels der Darstellung nach Hanes analysiert (A/V gegen A bei verschiedenen Werten von A, dabei ist A die Substratkonzentration und V die Reaktionsgeschwindigkeit).

### Beispiel 59 (nicht erfindungsgemäß)

### N-Acetyl-D-Glu-L-Cha-D-Pro-D-Tyr-L-Arg-amid

Dieses Peptid wurde wie in Beispiel 58 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 58 bestimmt und beträgt bei einer Peptidkonzentration von 1 µM 58%.

### Beispiel 60 (nicht erfindungsgemäß)

### N-Acetyl-D-Val-D-His-L-Cha-D-Pro-D-Tyr-L-Arg-amid

Dieses Peptid wurde wie in Beispiel 58 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 58 bestimmt und beträgt bei einer Peptidkonzentration von 1 µM 51%.

### Beispiel 61 (nicht erfindungsgemäß)

### N-Acetyl-L-Ala-L-Cha-D-Pro-D-Tyr-L-Arg-amid

Dieses Peptid wurde wie in Beispiel 58 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 58 bestimmt und beträgt bei einer Peptidkonzentration von 1 µM 44% .

### Beispiel 62 (nicht erfindungsgemäß)

### N-Acetyl-L-Ile-L-Arg-L-Cha-D-Pro-D-Tyr-L-Arg-amid

Dieses Peptid wurde wie in Beispiel 58 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 58 bestimmt und beträgt bei einer Peptidkonzentration von 1 µM 61%.

### Beispiel 63 (nicht erfindungsgemäß)

### N-Acetyl-L-Tyr-L-Cit-L-Cha-D-Pro-D-Tyr-L-Arg-amid

Dieses Peptid wurde wie in Beispiel 58 beschrieben synthetisiert und für die den Einsatz im Assay vorbereitet.

Die Hemmung des Thrombins wurde durch in-vitro-Hemmung der Amidase-Aktivität des Thrombins wie in Ausführungsbeispiel 58 bestimmt und beträgt bei einer Peptidkonzentration von 1 µM 55% .

### Beispiel 64 (nicht erfindungsgemäß)

### N-Acetyl-L-Ser-L-Ser-L-Cha-D-Pro-D-Tyr-L-Arg-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 1 µM 40%.

### Beispiel 65 (nicht erfindungsgemäß)

### N-Acetyl-D-Val-L-Cha-D-Pro-D-Tyr-L-Arg-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 1 µM 45% .

### Beispiel 66 (nicht erfindungsgemäß)

### N-Acetyl-L-Trp-L-Cha-D-Pro-D-Tyr-L-Arg-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 1 µM 63% .

### Beispiel 67 (nicht erfindungsgemäß)

### N-Acetyl-L-Ser-L-Ala-L-Cha-D-Pro-D-Tyr-L-Arg-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 1 µM 47%.

### Beispiel 68 (nicht erfindungsgemäß)

### N-Acetyl-L-Ser-L-Arg-L-Cha-D-Pro-D-Tyr-L-Arg-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 1 µM 51%.

### Beispiel 69 (nicht erfindungsgemäß)

### N-Acetyl-D-Lys-L-Nle-L-Cha-D-Pro-D-Tyr-L-Arg-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptid-konzentration von 1 µM 48%.

### Beispiel 70 (nicht erfindungsgemäß)

### N-Acetyl-D-Tyr-L-Cha-D-Pro-D-Tyr-L-Arg-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 1 µM 47%.

### Beispiel 71

### N-Acetyl-L-Arg-L-Cha-D-Pro-D-Tyr-L-Arg-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 1 µM 47%.

### Beispiel 72 (nicht erfindungsgemäß)

### N-Acetyl-L-Tyr-D-Pro-L-Cha-D-Pro-D-Tyr-L-Arg-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 1 µM 44% .

### Beispiel 73 (nicht erfindungsgemäß)

### N-Acetyl-L-Ala-D-Cha-L-Aze-D-Tyr-L-Arg-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 1 µM 63%.

### Beispiel 74 (nicht erfindungsgemäß)

### N-Acetyl-L-Ala-D-Cha-L-Pro-D-Tyr-L-Har-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 1 µM 56%.

### Beispiel 75 (nicht erfindungsgemäß)

### N-Acetyl-L-Trp-D-Cha-L-Aze-D-Tyr-L-Har-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 250 nM 76%.

### Beispiel 76 (nicht erfindungsgemäß)

### N-Acetyl-L-Ala-D-Cha-L-Aze-D-Tyr-L-Har-amid

Dieses Peptid wurde wie in Beispiel. 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 250 nM 77%.

### Beispiel 77 (nicht erfindungsgemäß)

### N-Acetyl-D-Phe-D-Cha-L-Aze-D-Tyr-L-Har-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 250 nM 77% .

### Beispiel 78 (nicht erfindungsgemäß)

### N-Acetyl-L-Dcp-D-Cha-L-Aze-D-Tyr-L-Har-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 250 nM 75% .

### Beispiel 79 (nicht erfindungsgemäß)

### N-Acetyl-L-Nhm-D-Cha-L-Aze-D-Tyr-L-Har-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 250 nM 80%.

### Beispiel 80 (nicht erfindungsgemäß)

### N-Acetyl-L-Iq3-D-Cha-L-Aze-D-Tyr-L-Har-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 250 nM 72% .

### Beispiel 81 (nicht erfindungsgemäß)

### N-Acetyl-L-Ppd-D-Cha-L-Aze-D-Tyr-L-Har-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptid-konzentration von 250 nM 76%.

### Beispiel 82 (nicht erfindungsgemäß)

### N-Acetyl-L-Tea-D-Cha-L-Aze-D-Tyr-L-Har-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 250 nM 74% .

### Beispiel 83 (nicht erfindungsgemäß)

### N-Acetyl-L-Phg-D-Cha-L-Aze-D-Tyr-L-Har-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 250 nM 95%.

### Beispiel 84 (nicht erfindungsgemäß)

### N-Acetyl-L-Nle-D-Cha-L-Aze-D-Tyr-L-Har-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 250 nM 89%.

### Beispiel 85 (nicht erfindungsgemäß)

### N-Acetyl-L-Cha-D-Cha-L-Aze-D-Tyr-L-Har-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 250 nM 90%.

### Beispiel 86 (nicht erfindungsgemäß)

### N-Acetyl-L-Pnp-D-Cha-L-Aze-D-Tyr-L-Har-amid

Dieses Peptid wurde wie in Beispiel 58 beschrieben synthetisiert und für die den Einsatz im Assay vorbereitet.
Die Hemmung des Thrombins wurde durch in-vitro-Hemmung der Amidase-Aktivität des Thrombins wie in Ausführungsbeispiel 58 bestimmt und beträgt bei einer Peptidkonzentration von 250 nM 72%.

### Beschreibung der Abkürzungen

- Ala: = Alanin Val = Valin
- Leu: = Leucin
- Ile: Isoleucin
- Pro =: Prolin
- Phe: = Phenylalanin
- Phg: = Phenylglycin
- Cha =: Cyclohexylalanin
- Trp: Tryptophan
- Met: = Methionin
- Gly: = Glycin
- Ser: = Serin
- Thr: = Threonin
- Cys: = Cystein
- Tyr: = Tyrosin
- Asn: = Asparagin
- Gln: = Glutamin
- Asp: = Asparaginsäure
- Glu: = Glutaminsäure
- Lys: = Lysin
- Arg: = Arginin
- His: = Histidin
- Nle: = Norleucin
- Orn: = Ornithin
- Cit: = Citrullin
- Aze: = Azetidin
- Har: = Homoarginin
- Dcp: = Dichlorphenylalanin
- Nhm =: Tetrahydronorhaman-3-carboxylsäure
- Iq3: = Tetrahydroisoquinolin-(1,2,3,4)-3-carboxylsäure
- Ppd: = 4-Phenylpiperidin-4-carbonsäure
- Tea: = Thienylalanin
- Pnp: = Paranitrophenylalanin

## Patentansprüche

1. Eine Verbindung der Formel (I)
Y¹-(NH-X¹-C=O)-(N-H-X²-C=O)-(NH-X³-C=O)-(NH-X⁴-C=O)-(NH-X⁵-C=O)-(NH-X⁶-C=O)-Y² (I),
wobei Y¹ entweder
a) ein Wasserstoff oder
b) eine Methylgruppe oder
c) eine Acetylgruppe ist oder
d) durch ein Rückgrat aus einer Kette von 1 bis 32 Kohlenstoffatomen charakterisiert ist,
wobei (NH-X¹-C=O) ein basischer Aminosäurebaustein, insbesondere
a) L-Arginin oder
b) D-Arginin oder
c) L-Lysin oder
d) D-Lysin oder
e) L-Ornithin oder
f) D-Ornithin ist,
wobei (NH-X²-C=O) eine cyclische, unpolare Aminosäure, insbesondere
a) L-Cyclohexylalanin oder
b) D-Cyclohexylalanin oder
c) L-Cyclohexylglycin oder
d) D-Cyclohexylglycin ist,
wobei (NH-X³-C=O) eine beliebige D- oder L-Aminosäure, insbesondere
a) L-Norleucin oder
b) D-Norleucin oder
c) L-Leucin oder
d) D-Leucin oder
e) L-Isoleucin oder
f) D-Isoleucin oder
g) L-Cyclohexylalanin oder
h) D-Cyclohexylalanin oder
i) L-Cyclohexylglycin oder
j) D-Cyclohexylglycin oder
k) L-Prolin oder
l) D-Prolin oder
m) L-Asparaginsäure oder
n) D-Asparaginsäure oder
o) L-Glutaminsäure oder
p) D-Glutaminsäure ist,
wobei (NH-X⁴-C=O) eine cyclische Aminosäure, insbesondere
a) L-Cyclohexylalanin oder
b) D-Cyclohexylalanin oder
c) L-Cyclohexylglycin oder
d) D-Cyclohexylglycin oder
e) L-Tyrosin oder
f) D-Tyrosin oder
g) L-Phenylalanin oder
h) D-Phenylalanin ist,
wobei (NH-X⁵-C=O) eine Aminosäure mit polarer Seitenkette, insbesondere
a) L-Glutamin oder
b) D-Glutamin oder
c) L-Ornithin oder
d) D-Ornithin oder
e) L-Glutaminsäure oder
f) D-Glutaminsäure oder
g) L-Arginin oder
h) D-Arginin oder
i) L-Lysin oder
j) D-Lysin oder
k) L-Asparagin oder
l) D-Asparagin oder
m) L-Asparaginsäure oder
n) D-Asparaginsäure ist oder
o) durch eine chemische Bindung ersetzt wird,
wobei (NH-X⁶-C=O) eine beliebige D- oder L-Aminosäure ist, insbesondere
a) L-Arginin oder
b) D-Arginin oder
c) durch eine chemische Bindung ersetzt wird,
wobei Y² entweder
a) eine OH-Gruppe (die C-endständige Aminosäure hat eine endständige Carbonsäure-Gruppe) oder
b) eine Aminogruppe (bei der C-endständigen Aminosäure ist die Carbonsäuredurch eine Amid-Gruppe ersetzt) oder
c) ein Wasserstoff (bei der C-endständigen Aminosäure ist die Carbonsäuredurch eine Aldehyd-Gruppe ersetzt) oder
d) 7-Amido-4-methylcumarin oder (über die Carbonsäuregruppe kombiniert) oder
e) Paranitroanilid (über die Carbonsäuregruppe kombiniert) oder
f) durch eine Verbindungskette aus 1 bis 35 Atomen ersetzt ist,
oder ein am C-Terminus und/oder am N-Terminus um nicht weniger als eine Aminosäure verkürztes Molekül, und pharmazeutisch akzeptable Salze davon.

2. Arzneimittel, **gekennzeichnet durch** seinen Gehalt an einer Verbindung nach Anspruch 1 neben üblichen Trägerstoffen, Hilfsmitteln und/oder Zusatzstoffen.

3. Diagnostische Zusammensetzung, **gekennzeichnet durch** ihren Gehalt an einer Verbindung nach Anspruch 1.

## Claims

1. A compound of the formula (I)
Y¹-(NH-X¹-C=O)-(NH-X²-C=O)-(NH-X³-C=O)-(NH-X⁴-C=O)-(NH-X⁵-C=O)-(NH-X⁶-C=O)-Y² (I),
wherein Y¹ is either
a) a hydrogen or
b) a methyl group or
c) an acetyl group or
d) is **characterised by** a backbone consisting of a chain of 1 to 32 carbon atoms,
wherein (NH-X¹-C=O) is a basic amino acid component, in particular
a) L-arginine or
b) D-arginine or
c) L-lysine or
d) D-lysine or
e) L-ornithine or
f) D-ornithine,
wherein (NH-X²-C=O) is a cyclic, non-polar amino acid, in particular
a) L-cyclohexylalanine or
b) D-cyclohexylalanine or
c) L-cyclohexylglycine or
d) D-cyclohexylglycine,
wherein (NH-X³-C=O) is any D-amino acid or L-amino acid, in particular
a) L-norleucine or
b) D-norleucine or
c) L-leucine or
d) D-leucine or
e) L-isoleucine or
f) D-isoleucine or
g) L-cyclohexylalanine or
h) D-cyclohexylalanine or
i) L-cyclohexylglycine or
j) D-cyclohexylglycine or
k) L-proline or
l) D-proline or
m) L-aspartic acid or
n) D-aspartic acid or
o) L-glutamic acid or
p) D-glutamic acid,
wherein (NH-X⁴-C=O) is a cyclic amino acid, in particular
a) L-cyclohexylalanine or
b) D-cyclohexylalanine or
c) L-cyclohexylglycine or
d) D-cyclohexylglycine or
e) L-tyrosine or
f) D-tyrosine or
g) L-phenylalanine or
h) D-phenylalanine,
wherein (NH-X⁵-C=O) is an amino acid with a polar side chain, in particular
a) L-glutamine or
b) D-glutamine or
c) L-ornithine or
d) D-ornithine or
e) L-glutamic acid or
f) D-glutamic acid or
g) L-arginine or
h) D-arginine or
i) L-lysine or
j) D-lysine or
k) L-asparagine or
l) D-asparagine or
m) L-aspartic acid
n) D-aspartic acid or
o) is replaced by a chemical bond,
wherein (NH-X⁶-C=O) is any D-amino acid or L-amino acid, in particular
a) L-arginine or
b) D-arginine or
c) is replaced by a chemical bond,
wherein Y² is either
a) an OH group (the C-terminal amino acid has a terminal carboxylic acid group) or
b) an amino group (in the C-terminal amino acid, the carboxylic acid is replaced by an amide group) or
c) a hydrogen (in the C-terminal amino acid, the carboxylic acid is replaced by an aldehyde group) or
d) 7-amido-4-methylcoumarin (combined through the carboxylic acid group) or
e) paranitroanilide (combined through the carboxylic acid group) or
f) is replaced by a connecting chain of 1 to 35 atoms,
or a molecule shortened at the C-terminus and/or at the N-terminus by not less than one amino acid, and pharmaceutically acceptable salts thereof.

2. A pharmaceutical agent, **characterised by** its content of a compound according to claim 1, apart from conventional carrier materials, auxiliaries and/or additives.

3. A diagnostic composition, **characterised by** its content of a compound according to claim 1.

## Revendications

1. Composé de formule (I)
Y¹-(NH-X¹-C=O)-(NH-X²-C=O)-(NH-X³-C=O)-(NH-X⁴-C=O)-(NH-X⁵-C=O)-(NH-X⁶-C=O)-Y² (I),
dans laquelle Y¹ soit représente
a) un atome d'hydrogène,
b) un groupe méthyle ou
c) un groupe acétyle, soit
d) est **caractérisé par** un squelette issu d'une chaîne de 1 à 32 atomes de carbone,
dans laquelle (NH-X¹-C=O) est un motif acide aminé basique, en particulier
a) la L-arginine,
b) la D-arginine,
c) la L-lysine,
d) la D-lysine,
e) la L-ornithine ou
f) la D-ornithine,
dans laquelle (NH-X²-C=O) est un acide aminé apolaire cyclique, en particulier
a) la L-cyclohexylalanine,
b) la D-cyclohexylalanine,
c) la L-cyclohexylglycine ou
d) la D-cyclohexylglycine,
dans laquelle (NH-X³-C=O) est un acide aminé D ou L quelconque, en particulier
a) la L-norleucine,
b) la D-norleucine,
c) la L-leucine,
d) la D-leucine,
e) la L-isoleucine,
f) la D-isoleucine,
g) la L-cyclohexylalanine,
h) la D-cyclohexylalanine,
i) la L-cyclohexylglycine,
j) la D-cyclohexylglycine,
k) la L-proline,
l) la D-proline,
m) l'acide L-aspartique,
n) l'acide D-aspartique,
o) l'acide L-glutamique ou
p) l'acide D-glutamique,
dans laquelle (NH-X⁴-C=O) est un acide aminé cyclique, en particulier
a) la L-cyclohexylalanine,
b) la D-cyclohexylalanine,
c) la L-cyclohexylglycine,
d) la D-cyclohexylglycine,
e) la L-tyrosine,
f) la D-tyrosine,
g) la L-phénylalanine ou
h) la D-phénylalanine,
dans laquelle (NH-X⁵-C=O) est un acide aminé possédant une chaîne latérale polaire, en particulier
a) la L-glutamine,
b) la D-glutamine,
c) la L-ornithine,
d) la D-ornithine,
e) l'acide L-glutamique,
f) l'acide D-glutamique,
g) la L-arginine,
h) la D-arginine,
i) la L-lysine,
j) la D-lysine,
k) la L-asparagine,
l) la D-asparagine,
m) l'acide L-aspartique ou
n) l'acide D-aspartique, ou bien
o) est substitué par une liaison chimique,
dans laquelle (NH-X⁶-C=O) est un acide aminé D ou L quelconque, en particulier
a) la L-arginine ou
b) la D-arginine, ou bien
c) est substitué par une liaison chimique,
dans laquelle Y² soit représente
a) un groupe OH (l'acide aminé C-terminal possède un groupe acide carboxylique terminal) ou
b) un groupe amino (dans l'acide aminé C-terminal, l'acide carboxylique est substitué par un groupe amide) ou
c) un atome d'hydrogène (dans l'acide aminé C-terminal, l'acide carboxylique est substitué par un groupe aldéhyde) ou
d) la 7-amido-4-méthylcoumarine (combinée avec le groupe acide carboxylique) ou
e) le paranitroanilide (combiné avec le groupe acide carboxylique), soit
f) est substitué par une chaîne de liaison de 1 à 35 atomes,
ou bien une molécule raccourcie d'un nombre pas inférieur à un acide aminé à l'extrémité C-terminale et/ou à l'extrémité N-terminale, et ses sels pharmaceutiquement acceptables.

2. Médicament **caractérisé par** sa teneur en un composé selon la revendication 1 en plus de véhicules, d'adjuvants et/ou d'additifs usuels.

3. Composition diagnostique **caractérisée par** sa teneur en un composé selon la revendication 1.
